# EUROPEAN PATENT APPLICATION

(11) **EP 3 838 329 A2**
(43) Date of publication of application: **23.06.2021**
(21) Application number: 20210749.6
(22) Date of filing: 30.11.2020
(51) Int. Cl.: A61N 1/04

(54) **ADDITIVE MANUFACTURING METHOD FOR MULTIDIRECTIONAL ELECTRODES**

(30) Priority: 19.12.2019 DE 102019220269
(71) Applicant: Heraeus Deutschland GmbH & Co KG, 63450 Hanau (DE)
(72) Inventor: Jung, Markus, 63450 Hanau (DE); Abu Asal, Murad, 63450 Hanau (DE); Hausch, Ulrich, 63450 Hanau (DE)
(74) Representative: Heraeus IP

(57) **Abstract**

The invention relates to a method for manufacturing a medical electrode, comprising the following steps: (i) providing an electrically insulating substrate material (111), on which a conductor track (112) is arranged; (ii) applying a continuous metal layer (113), which at least partially covers the substrate material (111), and the conductor track (112), so that an electrically conducting connection is formed between the metal layer (113) and the conductor track (112); and (iii) partially removing the metal layer (113) to form an electrode segment (114), which has an electrically conducting connection to the conductor track (112).

## Description

### FIELD OF THE INVENTION

The invention lies in the field of medical technology and relates to an additive manufacturing method for multidirectional electrodes, as well as an electrode manufactured by means of a method of this type.

### TECHNICAL BACKGROUND

Methods for manufacturing multidirectional electrodes, which can contain, for example, conductor structures of cermet, with the help of an HTCC method, also referred to as High-Temperature-Cofired-Ceramics methods in the technical field, are known from the prior art, such as, for example, from EP3406295A1. Several ceramic green sheets are applied one of top of the other thereby and are subsequently sintered at high temperature, for example at 1600°C to 1800°C. It is possible thereby to manufacture very small component structures comprising, for example, conductor tracks with particularly small dimensions, whereby the electrodes obtained thereby are very stable and resilient. However, there is a desire in the technical field to be able to miniaturize structures of this type even more, in order to provide for a smaller size of the electrode and/or a higher integration density of the obtained conductor structures.

### PREFERRED EMBODIMENTS

It is the object of the present invention to solve one or several of the above-described and further objects of the prior art. The invention will be described in an exemplary manner below by means of the following preferred embodiments. However, it is not limited to these embodiments.
1. A method for manufacturing a medical electrode, comprising the following steps:
   (i) providing an electrically insulating substrate material (111), on which a conductor track (112) is arranged,
   (ii) applying a continuous metal layer (113), which at least partially covers the substrate material (111), and the conductor track (112), so that an electrically conducting connection is formed between the metal layer (113) and the conductor track (112),
   (iii) partially removing the metal layer (113) to form an electrode segment (114), which has an electrically conducting connection to the conductor track (112).
2. The method according to embodiment 1, wherein the insulating substrate material comprises a ceramic or consists of a ceramic.
3. The method according to any one of the preceding embodiments, wherein the conductor track comprises a cermet or consists thereof.
4. The method according to any one of the preceding embodiments, wherein the application of a continuous metal layer comprises printing, spraying, PVD, dip coating, paste dosage, or ink deposition.
5. The method according to any one of the preceding embodiments, wherein the partial removal of the metal layer comprises laser ablation.
6. The method according to any one of the preceding embodiments, wherein several electrode segments (114), which are connected in an electrically conducting manner to different conductor tracks, are formed by the partial removal of the metal layer.
7. The method according to any one of the preceding embodiments, wherein the electrically insulating substrate material is provided as a plurality of layers (115), wherein at least one of the layers comprises a depression (116) for receiving an electrically conductive material.
8. The method according to embodiment 7, wherein the height of the depression (116) is smaller than the thickness (117) of a layer (115), wherein the thickness (117) of a layer (115) and the height of the depression (116) are defined along a common axis.
9. The method according to any one of the preceding embodiments, wherein a conductor track has a first portion (119) and a second portion (120), wherein the first portion is arranged at an angle α of 45° to 135°, preferably 60° to 120°, more preferably 80° to 110°, to the second portion.
10. A medical electrode, manufactured according to a method according to any one of the preceding embodiments.
11. A medical electrode having an electrically insulating substrate material (111) and a plurality of conductor tracks (112),
   characterized in
   that the smallest distance (121) between two adjacent conductor tracks (112) is smaller or larger than the height (122) of one of the two conductor tracks (112), wherein the distance (121) and the height (122) are defined along a common axis.
12. The medical electrode according to embodiment 11, wherein the distance (121) between two adjacent conductor tracks (112) is smaller than 100 µm, preferably smaller than 70 µm, more preferably smaller than 50 µm.
13. The medical electrode according to any one of embodiments 10 to 12, wherein the insulating substrate material comprises a ceramic or consists of a ceramic.
14. The medical electrode according to any one of embodiments 10 to 13, wherein at least one of the conductor tracks comprises a cermet or consists thereof.
15. The medical electrode according to any one of embodiments 10 to 14, further comprising several electrode segments (114), which are each connected in an electrically conducting manner to different conductor tracks (112).

### BRIEF DESCRIPTION OF THE DRAWINGS

Fig. 1 shows a cross section of an electrically insulating substrate material, on which a conductor track is arranged.
Fig. 2 shows a cross section of a continuous metal layer, which at least partially covers the substrate material and the conductor track.
Fig. 3 shows a cross section of a metal layer, which was partially removed to form an electrode segment.
Fig. 4 shows a top view from the top onto a metal layer, which was partially removed to form an electrode segment.
Fig. 5 shows a top view onto a precursor product for a metical electrode, which comprises several electrode segments, which are each electrically connected to another conductor track.
Fig. 6 shows a cross section of a layer of an electrically insulating substrate material, which has a depression.
Fig. 7 shows a cross section of a plurality of layers of an electrically insulating substrate material, wherein conductor tracks, which are formed by filling depressions with an electrically conductive material, are arranged between the layers.
Fig. 8 shows a cross section of a plurality of layers of an electrically insulating substrate material, wherein the height of a depression is smaller than the thickness of one of the adjacent layers.
Fig. 9 shows a cross section of a plurality of layers of an electrically insulating substrate material according to the prior art, wherein the height of a depression is equal to the thickness of the layer, in which the depression is located.
Fig. 10 shows a top view onto a section of a medical electrode, which has a conductor track, which has a first and a second portion, which are arranged at an angle to one another, which is 45° to 135°.
Fig. 11 shows a cross section of a section of a medical electrode, in the case of which the height of one of the two conductor tracks is smaller than the smallest distance between 2 adjacent conductor tracks.

### DETAILED DESCRIPTION OF THE INVENTION

### DEFINITIONS

In addition to the embodiments described herein, the elements of which "have" or "comprise" a certain feature (e.g. a material), a further embodiment is generally always considered, in which the respective element consists solely of the feature, i.e. does not comprise any further components. The word "comprise" or "comprising" is used synonymously with the word "have" or "having" herein.
When an element is identified in the singular form in an embodiment, an embodiment is likewise considered, in the case of which several of these elements are present. Unless otherwise specified or excluded unambiguously from the context, it is generally possible and is hereby unambiguously considered that features of different aspects and embodiments can also be present in the other aspects and embodiments described herein. It is likewise generally considered that all features, which are described herein in connection with a method, can also be used for the products described herein, for example for a precious metal complex compound and a composition, which includes such a precious metal complex compound, and vice versa. All of these considered combinations are not explicitly listed in detail in all cases only in the interest of a more concise description. Technical solutions, which are obviously equivalent to the features described herein, are to generally be comprised by the scope of the invention.

In the context of the invention, all composite substances of ceramic materials in a metallic matrix are referred to as "cermet" or as "containing cermet". They are characterized by a particularly high hardness and wear-resistance. The "cermets" and/or substances "containing cermet" are hard metal-related cutting materials, but which can manage without the hard substance tungsten carbide and which are manufactured by means of powder metallurgy. A cermet comprises a ceramic component and a metallic component. A sintering process for cermets and/or an electrode containing cermet runs in the same way as in the case of homogenous powders, in the case of which the metal is compacted more strongly than the ceramic with the same compressive force. Compared to sintered carbides, cermets have, for example, a higher thermo shock and oxidation resistance. The ceramic components of the cermet are, for example, aluminum oxide (AI203) and zirconium dioxide (ZrO2), while for example platinum, palladium, niobium, molybdenum, titanium, cobalt, zirconium, chromium are possible as metallic components.

The electrically insulating material is preferably a ceramic powder mass or paste, which has at least a minimal cohesion of the powder particles. This is usually realized in that a grain size of the powder particles does not exceed 0.5 mm. The manufacture of a green body thereby takes place, for example, by pressing powdered compositions or by formation and subsequent drying. For this purpose, a cermet-containing green body is created, for example, in parallel or subsequently. Several green bodies can be put together in a compound of a plurality of layers and can be fired subsequently.

"Firing" or "sintering" herein identifies a heat treatment below the melting temperature of the powder particles of the green body. This leads to a significant decrease of the porosity and of the volume of the green body.

### EXAMPLES

In a first aspect, a method for manufacturing a medical electrode is described, which comprises the following steps:
(i) providing an electrically insulating substrate material (111), on which a conductor track (112) is arranged,
(ii) applying a continuous metal layer (113), which at least partially covers the substrate material (111), and the conductor track (112), so that an electrically conducting connection is formed between the metal layer (113) and the conductor track (112),
(iii) partially removing the metal layer (113) to form an electrode segment (114), which has an electrically conducting connection to the conductor track (112).

The electrically insulating substrate material preferably comprises a ceramic. AI203 or ZrO2 are examples for a ceramic. In one embodiment, the insulating substrate material consists of a ceramic, for example aluminum oxide (Al2O3). In one embodiment, the electrically insulating substrate material comprises at least 95 percent by weight of aluminum oxide or at least 99 percent by weight of aluminum oxide.

The electrically insulating substrate material can be present in the form of a thin flat layer or ceramic film. The ceramic film can have a thickness of, for example, 0.05 to 1 mm, preferably 0.1 to 0.5 mm.

The conductor track comprises an electrically conductive material, for example a cermet or a metal. This metal can comprise, for example, platinum, gold, silver, niobium, tungsten, or tantalum, or alloys thereof. The cermet can comprise one of these metals and a ceramic, for example AI203 or ZrO2.
The cross-sectional surface of the conductor track can be, for example, 0.00008 to 0.008 mm², preferably 0.002 to 0.003 mm².

In a further embodiment, the conductor track comprises a cermet or consists of a cermet. A cermet comprises a ceramic material and a metallic material. An exemplary composition of a cermet is as follows:
45% by weight of Pt powder with a grain size of 50 µm,
45% by weight of AI203 powder with a grain size of 2 µm, and
10% by weight of binding agent, for example METAWAX P-50 by Zschimmer & Schwarz GmbH & Co. KG, or a similar binding agent. The relative percentages are each based on the total weight of the cermet.

For the manufacture of the Pt cermet, the Pt powder, the AI203 powder, and the binding agent can be mixed with AI203 balls on a roller block. The resulting mixture can then be filled into a double-Z-kneader and can be further mixed with a dispersing agent by adding demineralized water, in order to remove air pockets.

A continuous metal layer can be applied to the substrate material and to the conductor track, so that the substrate material and the conductor track are at least partially covered. As a result, the metal layer cohesively covers the surface in a flat manner, so that the metal layer has a good electrical conductivity, and an electrically conductive connection is established between the metal layer and the conductor track. The metal layer can comprise, for example, a metal selected from the group consisting of platinum, iridium, tantalum, palladium, titanium, iron, gold, molybdenum, niobium, tungsten, nickel, titanium, or alloys thereof, for example MP35, 316L, 301, 304, or combinations thereof. In one embodiment, the metal layer comprises platinum. In one embodiment, the metal layer consists of platinum.

The application of the metal layer can take place by means of any coating method, which is common in the technical field. In one embodiment, the application of a continuous metal layer comprises printing, spraying, PVD, dip coating, paste dosage, or ink deposition. The printing method can be, for example, a screen printing method or an inject printing method. The metal layer can also be applied by the separation from the gas phase, for example by means of physical vapor deposition (PVD). The metal layer can also be applied by means of sputtering or similar methods.

In that the metal layer is partially removed subsequently, one or several electrode segments can be formed from the metal layer. The electrode segments can be connected to a conductor track. The partial removal of the metal layer can take place by means of any suitable method. In one embodiment, the removal of the metal layer takes place by means of a material-selective method, i.e. the metal layer is removed, without the surrounding substrate material located underneath being removed or changed otherwise. The removal of the metal layer can take place mechanically, physically, or chemically.

The metal layer can be removed selectively, in order to give the metal layer a predetermined shape. In the alternative or in addition, the metal layer can also be applied with the help of a structuring method, for example by using a mask. The partial removal of the metal layer can take place, for example, by means of laser ablation in order to give a predetermined shape to the metal layer and to the electrode segment formed therefrom.
Optionally, several metal layers can be applied at various locations on the substrate material. For example, several conductor tracks can be present on the substrate material, and several or all conductor tracks can be provided with a respective metal layer, in order to manufacture an electrode segment at the desired location. It is possible thereby to manufacture an electrode comprising several, optionally individual electrode segments, which can be electrically addressed independently of one another. In one embodiment, several electrode segments, which are connected in an electrically conducting manner to different conductor tracks, are formed by means of the partial removal of the metal layer. In one embodiment, several electrode segments are formed from a single continuous, thus cohesive, metal layer, by means of partially removing this metal layer. In one embodiment, more than 50%, 60%, 70%, 80%, 90%, or more than 95% of the area of the metal layer is removed. In one embodiment, the removal of the metal layer takes place in one operating step, whereby several electrode segments are formed. In one embodiment, the formed electrode segments are electrically separated from one another, are thus not electrically connected to one another directly. In one embodiment, the electrode segments are arranged circumferentially along the surface of an electrode.
The electrode segments can generally have any geometry, for example rectangular, square, or round.

The above-described setup, which has an electrically insulating substrate material, on which a conductor track is arranged, can be manufactured as described below, before it is provided with the metal layer as described above:
In one embodiment, the electrically insulating substrate material is provided as a plurality of layers, whereby at least one of the layers comprises a depression for receiving an electrically conductive material.

The layers of the electrically insulating substrate material can be, for example, ceramic green films. The term "green film" can be understood to be a substrate layer and/or a film, which comprises a solid content, which can have organic material and inorganic material. The organic content among the solids can comprise, e.g., aluminum oxide, yttrium or scandium-stabilized zirconium oxide, or also other ceramic, metallic, or glassy materials, the inorganic content can additionally also comprise a corresponding amount of residual substances. The organic content can furthermore comprise binding agents, softening agents, and dispersing agents. Phthalates, polyols, glycols, phosphates, as well as other non-volatile hydrocarbons and the like can be used as softening agents. A manufacturing method of a ceramic green film is described, for example, in DE 19924134 A1. The green film can have a thickness of, for example, approximately 50 µm to 1000 µm, for example a thickness of approximately 100 to 500 µm, for example approximately 100 or 150 µm. In one example, the insulating substrate material has an aluminum oxide, i.e. AI203, in particular a ceramic slurry having aluminum oxide.
For example, a ceramic slurry having aluminum oxide can be used, as it is described, for example, in DE4016861 A1. With the help of further organic and inorganic additives, a fine-particle aluminum oxide powder is processed here into a casting compound, in order to manufacture a green film. An insulating layer of this type advantageously still has an insulating effect, even at a high temperature of above 1000°C. Due to the excellent mechanical properties, an insulating layer of this type is further particularly well suited for a co-sintering. An essentially crack-free structure can in particular be obtained with the use of a ceramic slurry of this type.

One or several of the layers of the electrically insulating material can be structured, for example with the help of mechanical processing or by means of laser ablation, such that a depression is formed therein. In one example, the mechanical processing of a layer of the electrically insulating material comprises laser ablation.
A depression in at least one of the plurality of layers can be set up to receive an electrically conductive material. In one example, the electrically conductive material comprises a cermet, as described herein.
In one example, an electrically conductive material is introduced into the depression. In one example, the introduction of the electrically conductive material into the depression takes place by means of a printing method or a dosing method (also referred to as dispensing in the technical field). In one example, the introduction of the electrically conductive material takes place by means of a screen printing method. The electrically conductive material introduced in the depression can form a conductor track. For this purpose, the depression can accordingly be formed geometrically. The depression can have, for example, an essentially elongated shape, i.e. the length of the depression is significantly larger than the smallest cross section of the depression. The cross-sectional surface of the depression can be, for example, 0.00008 to 0.008 mm², preferably 0.002 to 0.003 mm².

The plurality of layers, for example plurality of green films, can be formed into a compound by means of superposing. If necessary, the layers can be cut into the desired shape prior to the superposing. The individual layers can be aligned with one another prior to the superposing.
If the plurality of layers is a plurality of green films, the layer structure can be connected by means of pressing, for example isostatic pressing, and firing, to form a unit, in the case of which the layers can no longer be differentiated from one another after the firing. In a further example, the firing of the pressed layers has: firing the pressed plurality of layers at at least 1400°C for at least 36 hours.
The firing of the pressed layers can take place, for example, according to a method described in US 20040094417 A1 or in DE 19545590 C2.

After the firing, the sintered overall structure can be processed mechanically or with the help of laser cutting methods, in order to give a desired shape to the overall structure. In one example, accesses to the conductor tracks are formed by means of mechanical drilling or laser drilling. In another example, edges of the overall structure are rounded by means of laser ablation, so that it takes on, for example, the shape of a cylinder, which is rounded on one side or on both sides. For example, a rounded end of the overall structure can be provided with a metal layer, in order to form one or several electrode segments therefrom, as described herein.

In one example, the height of the depression is smaller than the thickness of a layer, wherein the thickness of a layer and the height of the depression are defined along a common axis. In other words, this means that the depression does not extend completely through the entire layer of the electrically insulating material, but only through a part of the layer. The depression is thus not continuous, but forms a groove or notch, for example. Compared to methods known in the prior art, the cross section of a conductor track can be decreased thereby, i.e. the diameter of a conductor track can, for example, be smaller than the layer thickness of a green film. The "thickness of a layer" refers, for example, to the thickness of a green film, whereby the decrease of the layer thickness through the depression is not included in the determination of the thickness. This means that the thickness of a layer is measured without consideration of the depression. The thickness of layer, for example of a ceramic green film, accordingly does not change in that a depression is introduced into the layer. If the layer is essentially plano-parallel, the thickness can thus be determined at a location other than the location of the depression. For example, the thickness of an essentially plano-parallel layer can be determined at the edge of this layer, even if the depression is located in the middle of the layer.

In one example, a conductor track has a first portion and a second portion, wherein the first portion is arranged at an angle of 45° to 135°, preferably 60° to 120°, more preferably 80° to 110°, to the second portion. This provides for a virtually arbitrary, i.e. almost completely flexible arrangement of the electrode segments on the surface of the electrodes, in particular in combination with the removal of the metal layer described therein, wherein each electrode segment can be connected to a different conductor track. For this purpose, the first portion of a conductor track can, for example, be arranged essentially parallel to the longitudinal axis of the electrode, while the second portion of a conductor track can be arranged at an angle, which differs therefrom, so that the second portion of the conductor track leads from the middle to the edge of the electrode, in order to provide for a contacting of an electrode segment. The first portion and the second portion can either directly adjoin one another and can form the above-mentioned angle with one another, or can be connected to one another via a curved segment. In one embodiment, the first portion and the second portion have an essentially linear shape, and are connected to one another via a curved segment of the conductor track. In this case, the above-mentioned angle is defined as the angle between the essentially linear first and second portions of the conductor track. This means that the angle between the 1. portion and the 2. portion is defined independently of the curve segment located therebetween.
In one embodiment, one conductor track has several curved portions. Such curved geometries can be provided in an advantageous manner, in that the depression in the insulating material is formed with the help of laser ablation, as described herein.

In a further aspect, a medical electrode is provided, which can be manufactured or is manufactured according to a method described herein.

In one example, the electrode has an electrically insulating substrate material and a plurality of conductor tracks, wherein the smallest distance between two adjacent conductor tracks is different from the height of one of the two conductor tracks, wherein the distance and the height are defined along a common axis.

Due to depressions, which have a smaller height than the layer thickness of the insulating layer, it is possible according to the invention to decrease the distance between two conductor tracks and/or the height of the conductor tracks compared to the prior art.

In one example, the electrode has an electrically insulating substrate material and a plurality of conductor tracks, wherein the smallest distance between two adjacent conductor tracks is smaller than the height of one of the two conductor tracks, wherein the distance and the height are defined along a common axis. Similarly as illustrated above with regard to the depression, this means that the distance between two conductor tracks can be particularly small compared to the prior art, because, when manufacturing the electrode, the cross section of the depression and of the electrically conductive material introduced therein can be smaller than the layer thickness of a green film, which defines the smallest possible distance between two conductor tracks in the prior art.

In one example, the electrode has an electrically insulating substrate material and a plurality of conductor tracks, wherein the smallest distance between two adjacent conductor tracks is larger than the height of one of the two conductor tracks, wherein the distance and the height are defined along a common axis.
In one example, the distance between two adjacent conductor tracks is smaller than 100 µm, preferably smaller than 70 µm, more preferably smaller than 50 µm.

In one example, the height of a conductor track is 0.05 to 1 mm; 0.1 to 0. 5 mm; 50 to 200 µm, or 10 to 100 µm.

In one example, the insulating substrate material comprises a ceramic, as described further above herein, or consists of a ceramic as described herein.

In one example, at least one of the conductor tracks comprises a cermet or consists thereof.

In one example, the electrode comprises several electrode segments, which are each connected in an electrically conducting manner to different conductor tracks.

In a further aspect, an electrical medical device is provided, having an electrode according to any one of the preceding aspects and embodiments thereof.

The electrical medical device can be, e.g., a lead, pulse generator, pacemaker, cardiac resynchronization device, sensor, or stimulator. Leads are electrical lines, which can be used, for example, in medical applications, such as neuromodulation, cardiac stimulation, deep brain stimulation, spinal cord stimulation, or gastric stimulation. In one embodiment, the lead is set up and/or intended to be connected to a generator of an active implantable device. In a medical device, a lead can also be used to receive an electrical signal. A stimulator is a medical device, which can achieve a physiological effect by emitting an electrical signal to the body of a living being. For example, a neurostimulator can effect an electrical signal in the nerve cell (e.g. an action potential) by emitting an electrical signal to a nerve cell.
A further embodiment relates to a microelectrode or a microelectrode array, which includes a system described herein.

A further aspect relates to a diagnostic method in or on the body of a living being, having the reception of an electrical signal by means of an electrode described herein. A further aspect relates to the use of an electrode described herein in a diagnostic method in or on the body of a living being, having the reception of an electrical signal by means of the electrode.
A further aspect relates to a therapeutic method in or on the body of a living being, having the emission of an electrical signal by means of an electrode described herein.

A further aspect relates to the use of an electrode described herein in a therapeutic method in or on the body of a living being, having the emission of an electrical signal by means of the electrode.
The therapeutic method can comprise the emission of an electrical signal to nerve cells or muscle cells in the region of an organ, for example heart, muscle, stomach, or brain. The diagnostic method can comprise the reception of an electrical signal from nerve cells or muscle cells in the region of an organ, for example heart, muscle, or brain.

### DETAILED DESCRIPTION OF THE DRAWINGS

The invention will be described in more detail below in a supplementary manner in several examples on the basis of the drawings. These examples serve to further clarify the invention. It is not intended that these examples are to limit the scope of protection of this patent application.

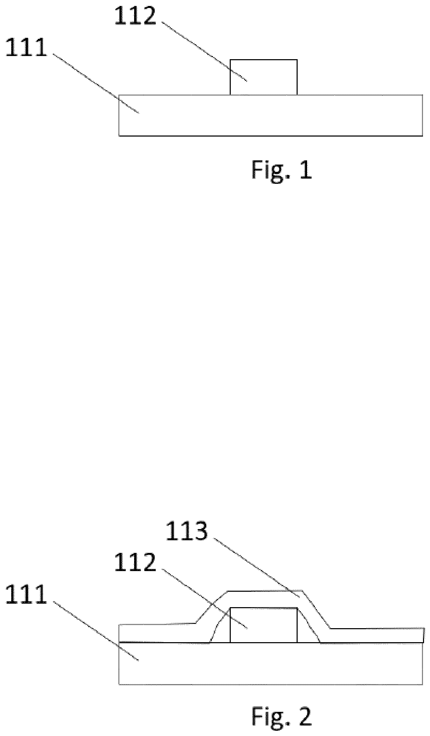

Fig. 1 shows a cross section of an electrically insulating substrate material 111, on which a conductor track 112 is arranged. The conductor track 112 can also be arranged in a depression of the substrate material 111, as it is described in more detail elsewhere herein. For example, the conductor track 112 can be arranged on the substrate material 111 such that the conductor track 112 does not form any protrusions whatsoever, which protrude beyond the surface plane of the substrate material 111. The conductor track 112 can be embedded into the substrate material 111. The conductor track 112 is electrically conductive and can comprise a metal or cermet. The conductor track 112 can comprise, for example, platinum or a cermet containing platinum.

Fig. 2 shows a cross section of a continuous metal layer 113, which at least partially covers the substrate material 111 and the conductor track 112. The metal layer can in each case completely or partially cover the substrate material 111 and/or the conductor track 112. In one embodiment, the continuous metal layer is more than 50% of the surface of the substrate material 111.

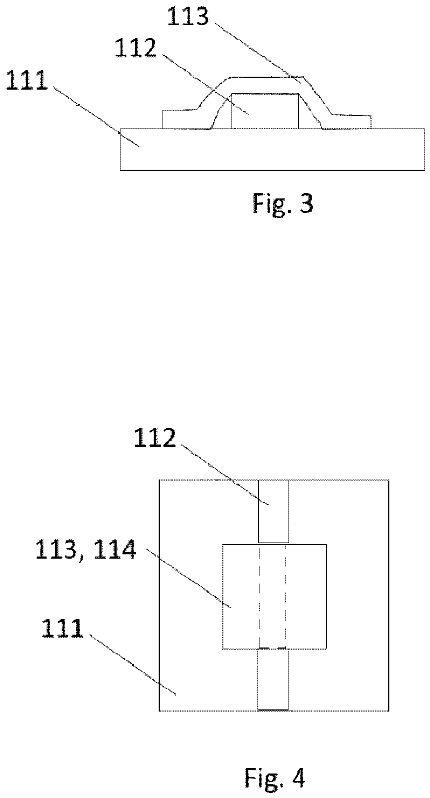

Fig. 3 shows a cross section of a metal layer 113, which was partially removed to form an electrode segment. This means that, after the partial removal, the metal layer 113 covers a smaller part of the surface of the substrate material 111 than prior to the removal of the metal layer 113. The metal layer 113, however, still at least partially covers the substrate material 111 and the conductor track 112 even after the removal, so that an electrically conducting connection still exists between the metal layer 113 and the conductor track 112. The metal layer 113 can also be removed in such a way that several electrode segments 114 are formed from a single metal layer 113.

Fig. 4 shows a top view from the top onto a metal layer 113, which was partially removed to form an electrode segment 114. The course of the conductor track 112 is illustrated by means of dashed lines. The electrode segment 114 is connected to a single conductor track 112, and only covers a part of the surface of the electrically insulating material 111.

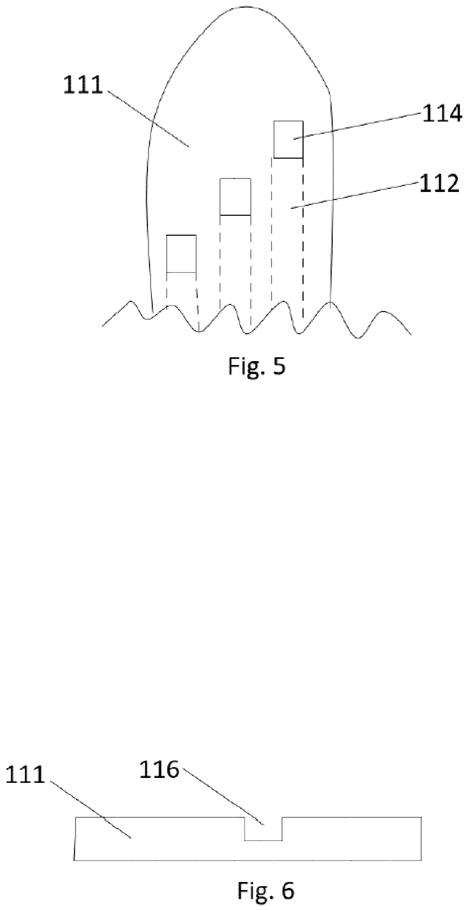

Fig. 5 shows a top view onto a precursor product for a medical electrode, which comprises several electrode segments 114, which are each electrically connected to another single conductor track 112, in each case illustrated by means of dashed lines. The individual electrode segments 114 can thus be electrically addressed independently of one another. The individual electrode segments 114 can be positioned largely independently of one another at virtually any locations of the electrode surface.

Fig. 6 shows a cross section of a layer of an electrically insulating substrate material 115, which has a depression 116. A depression 116 of this type can be set up to receive an electrically conductive material, in order to form a conductor track. This depression 116 can have, for example, the shape of a notch or groove. The electrically insulating substrate material can, for example, be a ceramic green film.

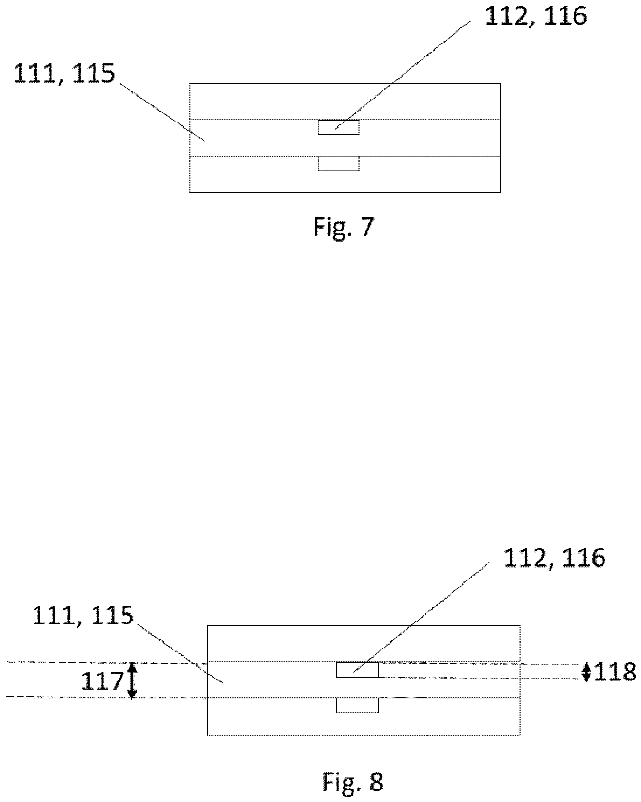

Fig. 7 shows a cross section of a plurality of layers 115 of an electrically insulating substrate material, wherein conductor tracks 112 are arranged between the layers 115, which are formed by filling depressions with an electrically conductive material, as descried above in Fig. 6. In this drawing, the plurality of layers 115 are arranged to form a common composite. This composite can obtain an improved cohesion of the individual layers by means of isostatic pressing. A composite can comprise several conductor tracks 112. The several conductor tracks 112 can be arranged within the same layer 115 and/or in different layers 115.

Fig. 8 shows a cross section of a plurality of layers of an electrically insulating substrate material, wherein the height 118 of a depression 116 is smaller than the thickness 117 of one of the adjacent layers 115 of the electrically insulating substrate material. The height 118 of the depression 116 and the thickness 117 of one of the adjacent layers 115 are defined along the same spatial direction, i.e. along axes, which are parallel to one another. In Fig. 8, these axes are illustrated as measuring bars. The thickness 117 of one of the adjacent layers 115 is thereby defined including the or independently of, respectively, the height 118 of a depression 116. This means that according to the definition, which applies herein, the thickness 117 of a layer 115 is independent of whether a depression is located therein or, in other words, the height of the depression does not impact the thickness of the layer. The thickness 117 of a layer 115 can, for example, be understood to be the thickness of a green film. In Fig. 8, the smallest distance between two depressions 116 is smaller than the thickness 117 of the layers 115, which include the depressions 116. The depressions 116, however, only extend partially, i.e. not completely, through one of the layers 115. The depressions 116 can be filled with an electrically conducting material, for example a cermet.

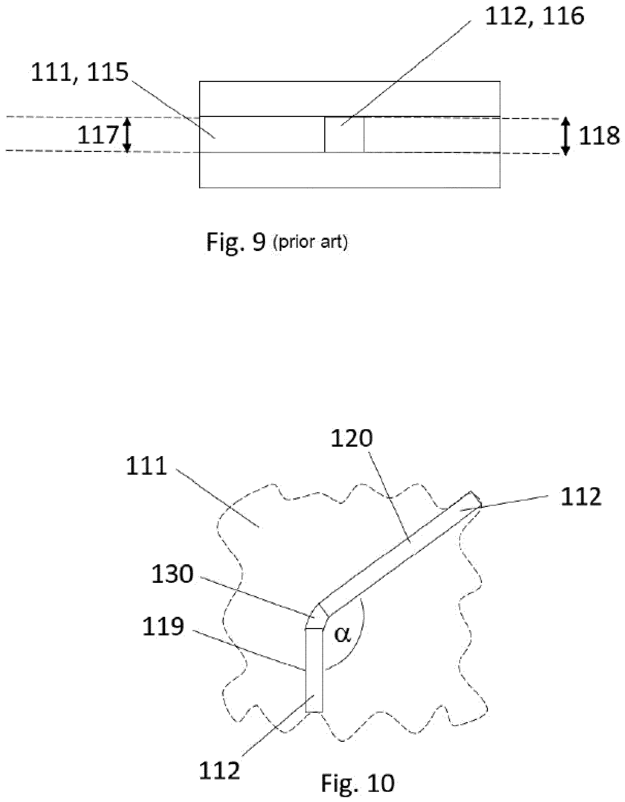

Fig. 9 shows a cross section of a plurality of layers of an electrically insulating substrate material according to the prior art, wherein the height 118 of a depression 116 is equal to the thickness 117 of a layer 115, in which the depression 116 is located. A depression 116 hereby extends completely through a layer 115.

Fig. 10 shows a top view onto a section of a medical electrode, which has a conductor track, which has a first and a second portion, which are arranged at an angle to one another, which is unequal to 180°, which is thus for example 45° to 135°. For example, the first portion 119 of a conductor track 112 can thereby be arranged essentially parallel to the longitudinal axis of the electrode, while the second portion 120 of a conductor track 112 is arranged at an angle α (alpha), which differs from 180°, so that the second portion 120 of the conductor track leads from the middle to the edge of the electrode in order to provide for a contacting of an electrode segment. The first portion 119 and the second portion 120 can either adjoin directly and can form the above-mentioned angle α with one another, or can be connected to one another via a curved segment 130. In one embodiment, the first portion 119 and the second portion 120 have an essentially linear shape, and are connected to one another via a curved segment 130 of the conductor track 112. In this case, the above-mentioned angle α (alpha) is defined as the angle between the essentially linear first 119 and second portions 120 of the conductor track 112. This means that the angle α between the 1. portion 119 and the 2. portion 120 is defined independently of the curve segment 130 located therebetween.

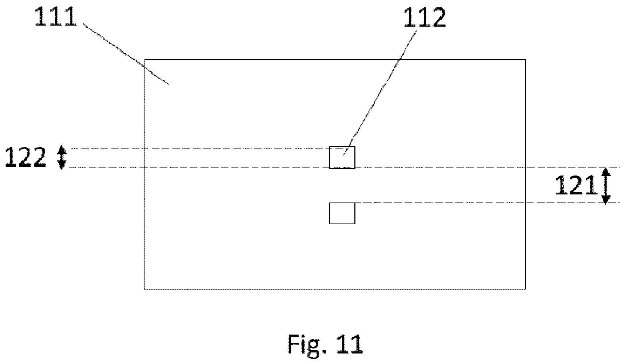

Fig. 11 shows a cross section of a section of a medical electrode, in the case of which the smallest distance 121 between 2 adjacent conductor tracks 112, which are arranged in the electrically insulating material 111, is larger than the height 122 of one of the two conductor tracks 112.

## Claims

1. A method for manufacturing a medical electrode, comprising the following steps:
(i) providing an electrically insulating substrate material (111), on which a conductor track (112) is arranged,
(ii) applying a continuous metal layer (113), which at least partially covers the substrate material (111) and the conductor track (112), so that an electrically conducting connection is formed between the metal layer (113) and the conductor track (112),
(iii) partially removing the metal layer (113) to form an electrode segment (114), which has an electrically conducting connection to the conductor track (112).

2. The method according to claim 1, wherein the insulating substrate material comprises a ceramic or consists of a ceramic.

3. The method according to any one of the preceding claims, wherein the conductor track comprises a cermet or consists thereof.

4. The method according to any one of the preceding claims, wherein the application of a continuous metal layer comprises printing, spraying, PVD, dip coating, paste dosage, or ink deposition.

5. The method according to any one of the preceding claims, wherein the partial removal of the metal layer comprises laser ablation.

6. The method according to any one of the preceding claims, wherein several electrode segments (114), which are connected in an electrically conducting manner to different conductor tracks, are formed by the partial removal of the metal layer.

7. The method according to any one of the preceding claims, wherein the electrically insulating substrate material is provided as a plurality of layers (115), wherein at least one of the layers comprises a depression (116) for receiving an electrically conductive material.

8. The method according to claim 7, wherein the height of the depression (116) is smaller than the thickness (117) of a layer (115), wherein the thickness (117) of a layer (115) and the height of the depression (116) are defined along a common axis.

9. The method according to any one of the preceding claims, wherein a conductor track has a first portion (119) and a second portion (120), wherein the first portion is arranged at an angle α of 45° to 135°, preferably 60° to 120°, more preferably 80° to 110°, to the second portion.

10. A medical electrode, manufactured according to a method according to any one of the preceding claims.

11. A medical electrode, having an electrically insulating substrate material (111) and a plurality of conductor tracks (112),
**characterized in that** the smallest distance (121) between two adjacent conductor tracks (112) is smaller or larger than the height (122) of one of the two conductor tracks (112), wherein the distance (121) and the height (122) are defined along a common axis.

12. The medical electrode according to claim 11, wherein the distance (121) between two adjacent conductor tracks (112) is smaller than 100 µm, preferably smaller than 70 µm, more preferably smaller than 50 µm.

13. The medical electrode according to any one of claims 10 to 12, wherein the insulating substrate material comprises a ceramic or consists of a ceramic.

14. The medical electrode according to any one of claims 10 to 13, wherein at least one of the conductor tracks comprises a cermet or consists thereof.

15. The medical electrode according to any one of claims 10 to 14, further comprising several electrode segments (114), which are each connected in an electrically conducting manner to different conductor tracks (112).
